# EUROPÄISCHE PATENTSCHRIFT

(11) **EP 0 301 323 B1**
(45) Veröffentlichungstag und Bekanntmachung des Hinweises auf die Patenterteilung: **12.05.1993**
(21) Anmeldenummer: 88111263.5
(22) Anmeldetag: 13.07.1988
(51) Int. Cl.: A61N 1/05, A61B 5/00, A61N 1/365

(54) **Katheter zur Implantation im Herz mit einer eingebauten Messsonde**
Catheter with a built-in probe for implantation in the heart
Cathéter à sonde de mesure intégrée pour implantation dans le coeur

(30) Priorität: 27.07.1987 DE 3724845
(43) Veröffentlichungstag der Anmeldung: 01.02.1989
(73) Patentinhaber: SIEMENS AKTIENGESELLSCHAFT, 80333 München (DE); Siemens-Elema AB, 171 95 Solna 1 (SE)
(72) Erfinder: Heinze, Roland, D-8000 München 80 (DE)

(56) Entgegenhaltungen:
- EP-A- 0 059 868
- DE-A- 2 142 983
- GB-A- 2 116 047
- US-A- 3 674 013
- US-A- 4 154 247
- US-A- 4 567 901
- US-A- 4 600 017

## Beschreibung

Die Erfindung betrifft einen Katheter mit eingebauter Meßsonde mit einer Vorkrümmung im Bereich der Meßsonde, wobei ein Meßfenster der Meßsonde an der Innenseite der Vorkrümmung angeordnet ist.

Ein derartiger Katheter ist aus der US-PS 3 674 013 bekannt. In dieser Patentschrift ist ein Katheter mit einer Glasfaseroptik zur Blutsauerstoffmessung in Blutgefäßen beschrieben. Um eine zu starke Annäherung des seitlich angeordneten Licht-Austrittsfensters der Glasfaseroptik an die Wände des Blutgefäßes zu vermeiden, weist der Katheter an seinem Ende eine Krümmung auf.

Katheter mit eingebauter Meßsonde zur Einführung in das Herz sind beispielsweise aus der DE-PS 31 52 963 bekannt. Dabei wird mit der Meßsonde die Blutsauerstoffsättigung nach dem Prinzip der Reflexionsoximetrie zur Frequenzregelung eines Herzschrittmachers gemessen. Über eine Leuchtdiode wird ebenfalls seitlich durch ein Fenster der Meßsonde Licht ausgestrahlt und mit einem Fototransistor das vom Blut in Abhängigkeit von dessen Sauerstoffsättigung reflektierte Licht gemessen. Diese Meßsonde kann nur befriedigende Meßergebnisse liefern, solange das Meßfenster, d.h. die aktive Fläche der Meßsonde nicht an der Wand, den Klappen oder Trabekeln des Herzens anliegt. Diese Einschränkung gilt nicht nur für die Blutsauerstoffmessung, sondern auch für andere Messungen, z.B. für die Impedanzmessung des Blutes. Der bekannte Katheter wird nach Einführung in das Herz leicht gekrümmt und es bleibt dem Zufall überlassen, ob das Meßfenster der Meßsonde an der Innen- oder Außenseite der Krümmung zu liegen kommt.

Aus der US-PS 4 567 901 ist ein Katheter mit Stimulationselektroden für Atrium und Ventrikel bekannt, bei dem der im Atrium liegende Teil eine Vorkrümmung aufweist, um eine Positionierung des Katheters zu erleichtern.

Aufgabe der Erfindung ist es, einen Katheter der eingangs genannten Art so auszuführen, daß die Meßsonde in einem definierten Bereich des Herz-Vorhofes und sicher vor Anlagerungen positioniert wird.

Diese Aufgabe wird erfindungsgemäß dadurch gelöst, daß der Katheter zur Implantation im Herz vorgesehen ist und daß der Katheter im dem der Katheterspitze abgewandten Bereich vor der Meßsonde die Krümmung aufweist und der Abstand der Krümmung und der Meßsonde zur Katheterspitze so gewählt sind, daß die Meßsonde im implantierten Zustand zwischen rechter Herzwand und Herzklappenebene zu liegen kommt. Durch diese Anordnung wird sichergestellt, daß die Meßsonde im Vorhofbereich positioniert wird, wenn der Katheter eingeführt ist. Durch die Vorkrümmung wird ein Anlegen des Meßfensters der Meßsonde an eine Herzwand verhindert. Insbesondere die Messung der Blutsauerstoffsättigung ist im Vorhofbereich am zweckmäßigsten, da hierbei Reflexionsartefakte durch Herzklappen und Trabekel vermieden werden.

Es hat sich gezeigt, daß die Anordnung der Vorkrümmung vor der Meßsonde ein Anlegen des Meßfensters an die Herzklappen sicherer verhindert als eine Vorkrümmung nach der Meßsonde. Der in der eingangs genannten US-PS 3 674 013 beschriebene Katheter mit einer durchgehenden Krümmung im Bereich der Katheterspitze verhindert zwar sehr gut das Anlegen des Meßfensters an die Wände von Blutgefäßen. Bei Einsatz im Herz könnte sich jedoch das Meßfenster leicht an eine Herzklappe anlegen, wenn der Abstand zur Katheterspitze nicht die Sensorposition im Vorhof garantiert. Eine eindeutige, reflexionssichere Positionierung der Meßsonde in einem bestimmten Bereich des Ventrikels ist nicht möglich. Die Positionierung des Sensors im Vorhof etwa in der Mitte zwischen rechter Herzwand und Klappenebene ist deshalb von Vorteil, weil die Durchmischung des venösen Blutes aus der oberen und unteren Hohlvene im mittleren Vorhofbereich am besten ist. Es hat sich herausgestellt, daß durch die Vorkrümmung vor der Meßsonde, also im Bereich des Vorhofes, eine eindeutigere Positionierung der Meßsonde im Vorhof möglich ist. Vorkrümmungen zur Katheterspitze hin, also im Ventrikelbereich, können zum Einrollen des Katheters vor allem zur Pulmonalarterie hin führen, wodurch der Sensor in die Klappenebene gerät. Bei der hohen Elastizität implantabler Katheter ergibt sich gegenüber der US-PS 3 674 013 durch Ventrikel-Vorkrümmung kein ausreichendes Drehmoment im Vorhofbereich.

Bei Ausführungen, wo die Sonde direkt in der Krümmung liegt, kann die Sonde zu nahe an die Herzwand geraten, wo der Durchmischungsgrad des Blutes nicht so gut ist wie vor der Herzklappe.

Die Vorkrümmung kann vorteilhafterweise dadurch erzeugt werden, daß die mechanischen Anschlüsse der Meßsonde an den Katheter gegenüber der Sondenachse abgewinkelt sind.

In einer weiteren vorteilhaften Ausführungsform kann die Vorkrümmung dadurch erzeugt werden, daß eine Isolationsschicht des Katheters eine Vorkrümmung aufweist.

Damit bleibt die Vorkrümmung elastisch, was das Einführen des Katheters erleichtert. In einer weiteren vorteilhaften Ausführungsform eines Katheters mit einer z.B. gewendelten, elektrischen Zuleitung kann die Vorkrümmung auch dadurch erzeugt werden, daß die Zuleitung zur Meßsonde in gekrümmtem Zustand mit Kunststoff mit elastischem Formschluß umgossen ist.

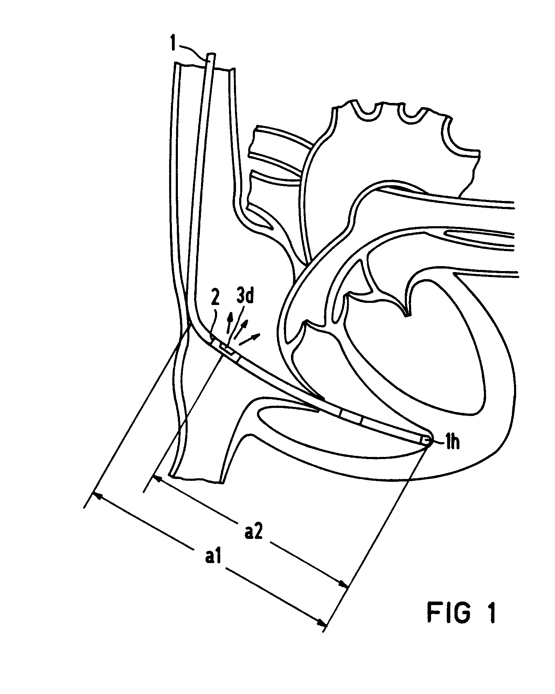

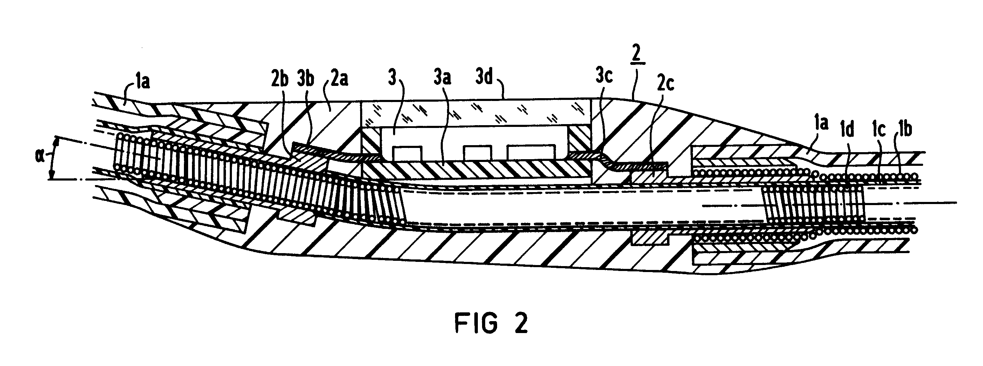

Ein Ausführungsbeispiel der Erfindung wird nachfolgend anhand der Figuren 1 und 2 näher erläutert.

Figur 1 zeigt die Anordnung eines in das Herz eingeführten Katheters 1 mit einer Stimulationselektrode 1h in der rechten Herzkammer. Im Vorhofbereich ist in den Katheter 1 eine Meßsonde 2 eingebaut, wobei der Katheter eine bei der Fertigung vorgegebene Vorkrümmung aufweist, so daß ein Anlegen des Meßfensters 3d an eine Herzwand mit dadurch bedingter Funktionsbeeinträchtigung vermieden wird. Die Vorkrümmung des Katheters optimiert die Positionierung der Meßsonde 2, indem sie sich der seitlichen Abwinkelung des Herzmuskels gegenüber dem Hauptvenenstrang natürlich anpaßt. Dies wird durch entsprechende Anpassung des Abstandes a1 der Krümmung und des Abstandes a2 der Meßsonde 2, jeweils von der Katheterspitze 1h an die Anatomie des Herzens erreicht.

Die Figur 2 zeigt ein Ausführungsbeispiel eines Katheters 1 mit Krümmung im Bereich vor der Meßsonde 2 anhand eines Schnittes in Längsrichtung des Katheters 1.

Der Katheter 1 ist in bekannter Weise mit zwei konzentrischen Drahtwendeln 1b und 1d aufgebaut, die durch eine innere Isolierung 1c voneinander isoliert sind. Der Katheter 1 ist von einer äußeren Isolierung 1a umschlossen. Die Meßsonde 2, die in diesem Fall der Erfassung der Blutsauerstoffsättigung dient, besteht aus dem Sondenkörper 2a mit den Anschlußstücken 2b und 2c sowie dem eigentlichen Meßsensor 3. Die Anschlußstücke 2b und 2c sind gegenüber der Sondenachse um α abgewinkelt. Die innere Drahtwendel 1d ist elektrisch mit dem Anschlußstück 2b der Meßsonde 2 verbunden und führt weiter zur Stimulationselektrode 1h. Die äußere Drahtwendel 1b ist elektrisch mit dem Anschlußstück 2c verbunden. Die äußere Isolierung 1a ist mechanisch fest mit der Meßsonde 2 verbunden derart, daß der Katheter 1 den gleichen Winkel gegenüber der Sondenachse hat wie die Anschlußstücke 2b und 2c.

Der Meßsensor 3 besteht hier aus einem Träger 3a für den optischen Halbleiter sowie der Glaslinse 3d als Meßfenster und den beiden elektrischen Anschlüssen 3b und 3c.

Zur Realisierung der Vorkrümmung des Katheters gibt es außer der Verwendung einer Meßsonde mit abgewinkelten Anschlüssen noch andere Möglichkeiten. Beispielsweise können eine oder beide Isolationsschichten 1a und 1c des Katheters 1 bei der Fertigung mit einer Vorkrümmung versehen werden. Eine Vorkrümmung ist auch dadurch möglich, daß eine oder beide Drahtwendel 1b, 1d in gekrümmtem Zustand mit Kunststoff mit elastischem Formschluß umgossen sind.

## Patentansprüche

1. Katheter mit eingebauter Meßsonde mit einer Vorkrümmung im Bereich der Meßsonde, wobei ein Meßfenster der Meßsonde an der Innenseite der Vorkrümmung angeordnet ist, **dadurch gekennzeichnet,** daß der Katheter zur Implantation im Herz vorgesehen ist und daß der Katheter in dem der Katheterspitze (1h) abgewandten Bereich vor der Meßsonde die Krümmung aufweist und der Abstand (a1) der Krümmung und der Abstand (a2) der Meßsonde (2) zur Katheterspitze (1h) so gewählt sind, daß die Meßsonde (2) im implantierten Zustand zwischen rechter Herzwand und Herzklappenebene zu liegen kommt.

2. Katheter nach Anspruch 1, **dadurch gekennzeichnet,** daß die Vorkrümmung dadurch erzeugt wird, daß die mechanischen Anschlüsse der Meßsonde (2) an den Katheter (1) gegenüber der Sondenachse abgewinkelt sind.

3. Katheter nach Anspruch 1 oder 2, **dadurch gekennzeichnet,** daß die Vorkrümmung dadurch erzeugt wird, daß eine Isolationsschicht des Katheters (1) eine Vorkrümmung aufweist.

4. Katheter nach Anspruch 1 oder 2 mit einem gewendelten Leiter als Zuleitung, **dadurch gekennzeichnet,** daß die Vorkrümmung dadurch erzeugt wird, daß die Zuleitung zur Meßsonde (2) in gekrümmtem Zustand mit Kunststoff mit elastischem Formschluß umgossen ist.

## Claims

1. Catheter with integral measuring probe having a preset curvature in the region of the measuring probe, with a measuring window of the measuring probe being arranged on the inside of the preset curvature, characterised in that the catheter is provided for implantation in the heart and in that the catheter has the curvature in the region, which is remote from the catheter tip (1h), in front of the measuring probe, and the distance (a1) of the curvature and the distance (a2) of the measuring probe (2) to the catheter tip (1h) are selected so that the measuring probe (2) in the implanted state lies between right heart wall and heart valve plane.

2. Catheter according to claim 1, characterised in that the preset curvature is produced in that the mechanical connections of the measuring probe (2) to the catheter (1) are angled with respect to the probe axis.

3. Catheter according to claim 1 or 2, characterised in that the preset curvature is produced in that an insulation layer of the catheter (1) has a preset curvature.

4. Catheter according to claim 1 or 2 having a helical conductor as a feed line, characterised in that the preset curvature is produced in that the feed line to the measuring probe (2) is recast, in the curved state, with plastics material with elastic form-fit.

## Revendications

1. Cathéter à sonde de mesure incorporée et à précourbure dans la zone de la sonde de mesure, dans lequel une fenêtre de mesure est prévue sur le côté de la précourbure, caractérisé par le fait que le cathéter est prévu pour son implantation dans le coeur, et que le cathéter présente la courbure dans la zone éloignée de la pointe (Lh) du cathéter, à l'avant de la sonde de mesure, et la distance (a1) de la courbure et la distance (a2) entre la sonde de mesure (2) et la pointe du cathéter (1h) sont choisies de telle manière que le sonde de mesure (2) vient, à l'état implanté, à se situer entre la paroi cardiaque droite et le plan de valvules cardiaques.

2. Cathéter selon la revendication 1, caractérisé par le fait que la précourbure est réalisée grâce au fait que les branchements ou raccords mécaniques de la sonde de mesure (2) au cathéter (1) sont coudés par rapport à l'axe de la soude.

3. Cathéter selon la revendication 1 ou 2, caractérisé par le fait que la précourbure est produite du fait qu'une couche d'isolation du cathéter (1) présente une précourbure.

4. Cathéter selon la revendication 1 ou 2 possédant comme conducteur d'alimentation un fil boudiné (spiralé), caractérisé par le fait que la précourbure est produite grâce au fait que le conducteur d'alimentation de la sonde de mesure (2), à l'état courbé, est enrobée de matière plastique, avec liaison élastique par formes complémentaires.
